# EUROPEAN PATENT SPECIFICATION

(11) **EP 4 010 693 B1**
(45) Date of publication and mention of the grant of the patent: **27.09.2023**
(21) Application number: 20754393.5
(22) Date of filing: 06.08.2020
(51) Int. Cl.: G01N 31/12, F23C 3/00

(54) **OXY-PYROHYDROLYSIS REACTORS WITH PROTECTED INSERTS**
OXY-PYROHYDROLYSE-REAKTOREN MIT GESCHÜTZTEN EINSÄTZEN
RÉACTEURS D'OXY-PYROHYDROLYSE À INSERTS PROTÉGÉS

(30) Priority: 09.08.2019 US 201962885043 P
(43) Date of publication of application: 15.06.2022
(73) Proprietor: 3M Innovative Properties Company, Saint Paul, Minnesota 55133-3427 (US)
(72) Inventor: VORARATH, Phanasouk, Saint Paul, Minnesota 55133-3427 (US)
(74) Representative: Mathys & Squire
(86) International application number: PCT/IB2020/057456
(87) International publication number: WO 2021/028799

(56) References cited:
- WO-A1-2017/172390
- JP-A- S51 139 392
- JP-B1- S4 833 719

## Description

### FIELD

The present disclosure relates to oxy-pyrohydrolysis reactors with protected inserts. Methods for total halogen analysis, in particular, for fluorine content analysis, in various samples using such reactors are also described.

### SUMMARY

Briefly, in one aspect, the present disclosure provides an oxy-pyrohydrolysis reactor comprising: a pyrotube comprising a combustion chamber positioned between a first end and a second end the pyrotube; and a sample insert connected to the first end of the pyrotube. The sample insert comprises a sample insert tube that extends through the first end of the pyrotube such that it is located within the combustion chamber of the pyrotube a first distance X1 from the first end of the pyrotube, and the outer opening is located outside the pyrotube; and a protective tube connected to the sample insert tube extending a second distance X2 from the first end of the pyrotube into the combustion chamber of the pyrotube, wherein the ratio of X2 over X1 is at least 2. The sample insert tube comprises a first material selected from quartz, glass, and combinations thereof; and the protective tube comprises a second material selected from a ceramic, a metal, a metal alloy, and combinations thereof.

In another aspect, the present disclosure provides combustion systems including such oxy-pyrohydrolysis reactors.

In yet another aspect, the present disclosure provides methods using of such oxy-pyrohydrolysis reactors and systems. Such methods comprise delivering, via a sample insert, a sample into a pyrotube from a first end thereof, the sample containing one or more halogen elements; and combusting the sample inside the pyrotube to produce combustion products. In some embodiments, the methods further include analyzing the combustion products to determine the total halogen (e.g., fluorine) content of the sample.

Further details of other aspects and various embodiments of this disclosure are also set forth in the description below. Other features, objects, and advantages of the invention will be apparent from the description and from the claims.

### BRIEF DESCRIPTION OF THE DRAWINGS

**FIG. 1** is a schematic diagram of an analytical combustion system.
**FIGS. 2A - 2C** illustrate oxy-pyrohydrolysis reactors.
**FIG. 3** illustrates a prior art sample insert tube.
**FIG. 4** illustrates an exemplary sample insert tube according to some embodiments of the present disclosure.
**FIG. 5** illustrates another exemplary sample insert tube according to some embodiments of the present disclosure.
**FIG. 6** illustrates an exemplary oxy-pyrohydrolysis reactor including an exemplary sample insert tube according to some embodiments of the present disclosure.
**FIG. 7** illustrates another exemplary oxy-pyrohydrolysis reactor including an exemplary sample insert tube according to some embodiments of the present disclosure.

### DETAILED DESCRIPTION

Commercial analytical combustion systems are available and can be selected to target for halogens. Generally, samples containing halogens are combusted inside a pyrotube placed inside a furnace. Combustion products (e.g., gases) can be analyzed to determine the total content of one or more halogens in the samples. Various analytical techniques for determining element contents in samples are described in, for example, U.S. Patent Nos. 4,401,763 and 4,285,699.

Improved systems and techniques are disclosed in International Patent Publication WO 2017/172390 A1 ("Oxy-Pyrohydrolysis System and Method for Total Halogen Analysis," P. Vorarath). Those devices included a combustion-enhancing bed including ceramic fiber or fabrics disposed inside the pyrotube to enhance the combustion and protect the pyrotube from damage by corrosive gases.

Despite the advantages of those devices, the present inventors discovered additional improvements to oxy-pyrohydrolysis systems that may further increase the reliability of total halogen analysis and extend the life of critical components such as the pyrotube.

For example, although the ceramic fibers or fabrics protected portions of the pyrotube previously known to be affected by corrosion, the enhanced combustion and the associated increase in HF concentration introduced new regions of corrosion that may limit the life of such systems. Also, although the systems of WO 2017/172390 A1 could be used with a wide variety of samples, many desirable samples (e.g., blood, soil, and seawater) contain salts, minerals and other contaminants. The presence of these materials in combination with the enhanced combustion can further contribute to increased corrosion. In addition, as the pyrotubes and other components are typically made of quartz, corrosion or etching of these parts by HF or salts can release fluorine from the quartz itself, introducing an error in the measured concentrations. This may not be a significant source of error when high concentrations of halogens are present in the samples themselves but can be a problem when attempting to accurately measure lower concentrations, e.g., less than 500 ppm, less than 250 ppm, or even less than 100 ppm.

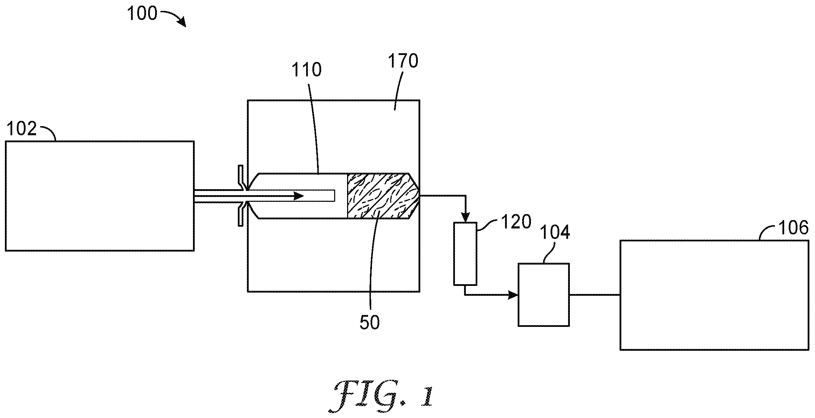

**FIG. 1** is a schematic diagram of analytical combustion system **100,** for example, a system for analyzing the total halogen content in samples, according to some embodiments of the present disclosure. Test samples (e.g., solids, liquids, emulsions, gases, etc.) can be introduced via sample introduction module **102** into oxy-pyrohydrolysis reactor **110** which can be, for example, a quartz pyrotube. In some embodiments, sample introduction module **102** may include a boat carrier. A known weight of a test portion of a sample can be carried by the boat carrier at a controlled rate into oxy-pyrohydrolysis reactor 110. In some embodiments, sample introduction module **102** may include a liquid delivery instrument such as, for example, a pump, an injector, etc., for continuously delivering liquid samples into the oxy-pyrohydrolysis reactor.

The oxy-pyrohydrolysis reactor is placed inside furnace **170,** which operates at high temperatures (e.g., 1000 °C to 1100 °C). Combustion ingredients (e.g., oxygen and/or water) can be provided into oxy-pyrohydrolysis reactor **110** to assist in burning the test samples at high temperatures. Under such oxy-pyrohydrolysis conditions (e.g., water, oxygen, heat, etc.), halogen elements (e.g., chlorine, bromine, fluorine, etc.) contained in the test sample can be converted into combustion products including gaseous compounds such as, for example, fluorides. In some embodiments, the combustion products can be trapped in a condensed steam or buffer inside condenser **120.**

An analyzer can be functionally connected to the furnace, e.g., through the condenser, and configured to analyze the composition of the combustion products. In some embodiments, liquid water containing halogen ions (e.g., fluoride ions) can be separated from gases at condenser **120** before it gets transferred, via pump **104,** to analyzer **106** where the total content of halogen elements (e.g., fluorine) can be analyzed. Suitable analyzers are known in the field and commercially available including, e.g., a fluoride meter module comprising, e.g., an anion chromatograph or an ion selective electrode. In some embodiments, system **100** can analyze the total fluorine content in a sample that is in the range, e.g., from about 0.005 wt% to about 35 wt%.

In some embodiments, combustion-enhancing bed **50** is provided inside oxy-pyrohydrolysis reactor **110.** The combustion-enhancing bed includes ceramic fibers or fabrics which can effectively enhance the combustion of samples inside reactor **110.**

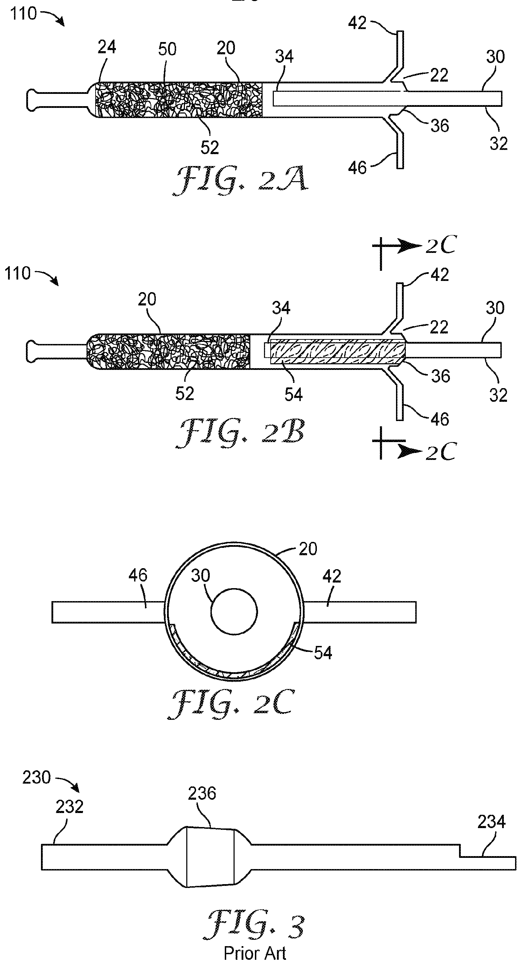

**FIGS. 2A-2C** illustrate various oxy-pyrohydrolysis reactors **110** and their components, according to various embodiments. Referring to **FIG. 2A****,** oxy-pyrohydrolysis reactor **110** includes pyrotube **20** extending along an axis thereof between first end **22** and second end **24** opposite the first end and defining a combustion chamber. Generally, pyrotube **20** is made of materials that can withstand high temperatures (e.g., about 1100 °C or higher), for example, quartz, glass, ceramics, and metals such as platinum. Typically, the pyrotube is made of quartz or glass, preferably quartz. The dimension of the pyrotube are not critical and may be selected to meet specific needs. In some embodiments, pyrotube **20** can have an internal diameter of, for example, about 10 mm to about 10 cm, and a length of, for example, about 10 cm to about 100 cm.

Fluid inlets **42** and **46** are located near first end **22** of pyrotube **20** and configured to direct combustion ingredients such as, for example, oxygen and water into the body of the pyrotube. It is to be understood that one or more of the combustion ingredients may be optional. For example, for testing liquid samples, additional water may not be needed.

Sample insert **30** extends between first end **32** and second end **34** thereof and is connected to first end **22** of the pyrotube **20** at a junction **36.** The sample insert is a separate component connected to the pyrotube. In some embodiments, the sample insert may have respective desirable structures according to the different type of samples (e.g., solids, liquids, emulsions, gases, etc.) to be delivered. For example, second end **34** may be configured to hold samples comprising a solid, a liquid, an emulsion, or a combination thereof. Alternatively, second end **34** may be configured to deliver fluid samples, e.g., liquids or gases.

In the depicted embodiment of **FIG. 2A****,** optional combustion-enhancing bed **50** is disposed inside pyrotube **20** and downstream of second end **34** of sample insert **30.** Combustion-enhancing bed **50** comprises ceramic fiber **52.** In some embodiments, ceramic fibers **52** include strings of ceramic fibers that are randomly rolled and packed inside the pyrotube. The packing density of the fibers can be suitable to allow combustion gases and steam vapor to pass through.

In some embodiments, the ceramic fibers can include, for example, alumina-based inorganic oxide fibers. The alumina-based inorganic oxide fibers typically have an average effective fiber diameter of at least about 5 micrometers, although this is not a requirement. In some embodiments, the average effective fiber diameter is less than or equal to 50 micrometers or less than or equal to 25 micrometers.

Useful alumina-based inorganic oxide fibers include, for example, aluminoborosilicate fibers as described in U.S. Patent No. 3,795,524 (Sowman). In some embodiments, the aluminoborosilicate fibers comprise, on a theoretical oxide basis: about 35 percent by weight to about 75 percent by weight (more preferably, about 55 percent by weight to about 75 percent by weight) of Al2O3; greater than 0 percent by weight (more preferably, at least about 15 percent by weight) and less than about 50 percent by weight (more preferably, less than about 45 percent, and most preferably, less than about 40 percent) of SiO2; and greater than about 1 percent by weight (more preferably, less than about 25 percent by weight, even more preferably, about 1 percent by weight to about 20 percent by weight, and most preferably, about 2 percent by weight to about 15 percent by weight) of B2O3, based on the total weight of the aluminoborosilicate fibers. Preferred aluminoborosilicate fibers are commercially available as NEXTEL 312 inorganic oxide fiber from 3M Company, Maplewood, Minnesota.

Useful alumina-based inorganic oxide fibers also include aluminosilicate fibers. Aluminosilicate fibers, which are typically crystalline, comprise aluminum oxide in the range from about 67 to about 97 percent by weight and silicon oxide in the range from about 3 to about 33 percent by weight. Aluminosilicate fibers can be made as disclosed, for example, in U.S. Patent No. 4,047,965 (Karst et al.). In some embodiments, the aluminosilicate fibers include, on a theoretical oxide basis, from about 67 to about 85 percent by weight of Al2O3 and from about 33 to about 15 percent by weight of SiO2, based on the total weight of the aluminosilicate fibers. In some embodiments, the aluminosilicate fibers include, on a theoretical oxide basis, from about 67 to about 77 percent by weight of Al2O3and from about 23 to about 33 percent by weight of SiO2, based on the total weight of the aluminosilicate fibers. In some embodiments, the aluminosilicate fiber includes, on a theoretical oxide basis, from about 85 to about 97 percent by weight of Al2O3 and from about 3 to about 15 percent by weight of SiO2, based on the total weight of the aluminosilicate fibers. Aluminosilicate fibers are commercially available, for example, as NEXTEL 550 and NEXTEL 720 aluminosilicate fiber from 3M Company.

In some embodiments, the alumina fibers include, on a theoretical oxide basis, greater than about 98 percent by weight of Al2O3 and from about 0.2 to about 1.0 percent by weight of SiO2, based on the total weight of the alumina fibers. Alpha alumina fibers are available, for example, as NEXTEL 610 inorganic oxide fiber from the 3M Company.

Referring now to **FIGS. 2B** and **2C****,** in some embodiments, sheet **54** of ceramic fabric or fiber is disposed in pyrotube **20** near first end **22** of the pyrotube. Sheet **54** is positioned between oxygen inlet **42** and water inlet **46.** Sheet **54** of ceramic fabric or fiber can help spread and quickly evaporate water from the water inlet along the pyrotube. The porosity of the fabric or fiber can spread the water preventing it from pooling. In some embodiments, sheet **54** can extend, for example, about one-quarter to about one-half of the length of the pyrotube. The ceramic fibers of sheet **54** can be the same or different from ceramic fibers **52** of **FIG. 2A****.**

**FIG. 3** is a cross sectional view of sample insert **230** of the prior art. Sample insert **230** extends from first end **232** to second end **234.** Sample insert **230** further includes junction **236** configured to allow sample insert **230** to be connected to the first end of a pyrotube. Generally, such pyrotubes are made of quartz or glass to allow easy formation of the structures and to facilitate connection to quartz pyrotubes.

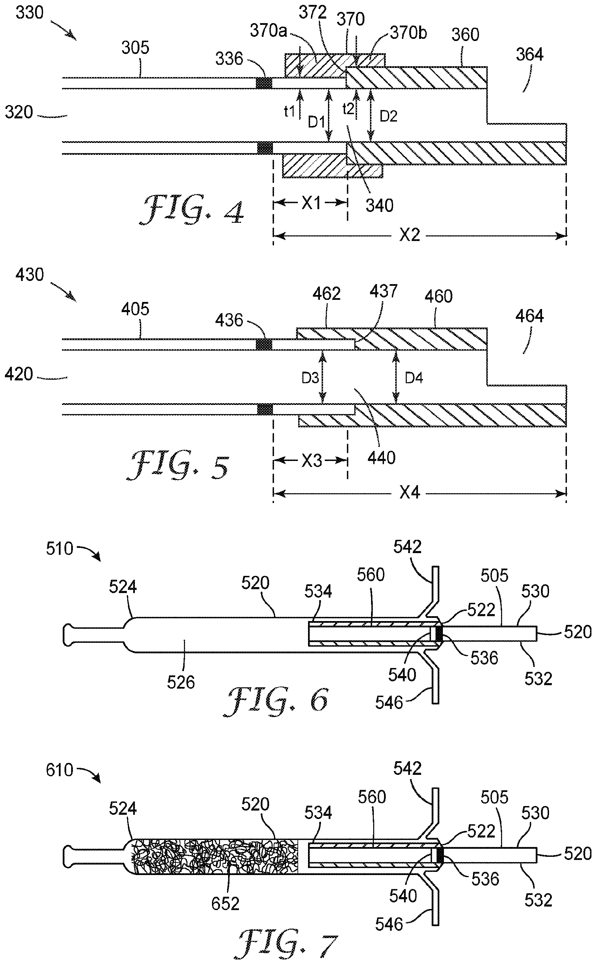

**FIG. 4** is a cross sectional view of exemplary sample insert **330,** according to some embodiments of the present disclosure. Sample insert **330** can be used in any oxy-pyrohydrolysis reactor including those described herein, and variations thereof. Sample insert **330** includes sample insert tube 305 having inner opening **340** and outer opening **320.**

Sample insert **330** further includes protective tube **360** connected to inner opening **340** of sample insert tube **305.** Generally, the connection between the protective tube and the inner opening of the sample insert tube is a sealed connection to eliminate leaks. Protective tube **360** includes second end **364,** serving as the inner opening of sample insert **330.** Second end **364** may be configured to hold samples to be inserted into the pyrotube, e.g., samples comprising solids, liquids, emulsions and combinations thereof. Second end **364** may also be configured to deliver fluid samples, e.g., liquid or gases to the desired location in the pyrotube.

When sample insert **330** is assembled with a pyrotube, the sample insert can be connected to the pyrotube at junction **336,** with sample insert tube **305** extending a distance **X1** into the pyrotube. In some embodiments, it can be desirable to minimize distance **X1** to limit the potential for exposure to the corrosive combustion environment.

When sample insert **330** is assembled with a pyrotube at junction **336,** protective tube **360** extends a distance **X2** into the combustion chamber of the pyrotube to position second end **364** at the desired location, wherein **X2** is greater than **X1**and the ratio of **X2** over **X1** is at least 2, e.g., at 10, at least 20, or even at least 50. In some embodiments, protective tube **360** may cover some of or all of the portion of sample insert tube **305** that extends into the combustion chamber.

Generally, sample insert tube **305** is made of materials that can withstand high temperatures (e.g., about 1100 °C or higher) selected from quartz or glass or combination thereof. In some embodiments, sample insert tube **305** is a quartz tube..

Generally, protective tube **360** is made of materials selected from a ceramic, a metal, a metal alloy, and combinations thereof that can withstand high temperatures, but also resist damage from corrosive gases produced in the combustion process, such as, for example, hydrogen fluoride and/or salts. Without protective tube **360,** the portion of sample insert tube **305** disposed inside a combustion chamber may suffer damage by such corrosive gases. In some embodiments, the protective tubes can withstand high temperatures (e.g., about 1000 °C, about 1100 °C, or even higher). In some embodiments, protective tube **360** may be made of materials having a melting point of about 1500 °C or higher and may not bend or soften at about 1000 °C to about 1100 °C or higher.

In some embodiments, the protective tube comprises a ceramic. In some embodiments, the protective tube can include one or more metals or metal alloys. In some embodiments, the protective tube can include platinum.

Referring to **FIG. 4****,** in some embodiments, protective tube **360** has an inner diameter **D2** substantially the same as an inner diameter **D1** of sample insert tube **305** at inner opening **340** thereof. In the depicted embodiment of **FIG. 4****,** sleeve **370** connects protective tube **360** to sample insert tube **305.** Sleeve **370** has first portion **370a** covering the outer surface of sample insert (or inner) tube **305** and second portion **370b** connected to protective tube **360.** In some embodiments, protective tube **360** has a thickness **t2** greater than the thickness **t1** of inner tube **305** at inner opening **340** thereof. Sleeve **370** may include step **372** between first and second portions **370a** and **370b** to accommodate the thickness difference of the inner tube and the protective tube.

Sleeve **370** can be connected to inner tube **305** and protective tube **360** by any suitable mechanisms. In some embodiments, sleeve **370** can be connected to the inner tube and the protective tube by, e.g., adhesives to form the sample insert. In some embodiments, the sleeve can be connected to the inner tube and the protective tube by, e.g., a ground joint or a threaded connection. Generally, the connections provide a seal to prevent leakage.

Sleeve **370** can be made of any suitable materials that can resist the corrosion of hydrogen fluoride and/or salts. In some embodiments, sleeve **370** can be a ceramic tube. In some embodiments, the sleeve can include one or more metals or metal alloys (e.g., Pt or its alloy).

**FIG. 5** is a cross sectional view of sample insert **430,** according to another embodiment. Sample insert **430** can be used in any oxy-pyrohydrolysis reactors described herein or variations thereof. Sample insert **430** includes sample insert tube **405** having inner opening **440** and outer opening **420.** Sample insert **430** includes protective tube **460** connected to inner opening **440** of sample insert tube **405.** When sample insert **430** is assembled with a pyrotube, the sample insert can be connected to the pyrotube at junction **436.** When assembled, inner opening **440** of sample insert tube **405** extends a distance **X3** into the combustion chamber, and second end **464** of protective tube **460** extends a distance **X4** into the combustion chamber of the pyrotube to form the inner opening of sample insert **430.** Generally, **X4** is greater than **X3.** The ratio of **X4** over **X3** is at least 2, e.g., at least 5, at least 10 or even at least 20.

In the embodiments, of **FIG. 5****,** protective tube **460** has an inner diameter **D4** substantially the same as an inner diameter **D3** of sample insert tube **405** at inner opening **440** thereof. In the depicted embodiment of **FIG. 5****,** protective tube **460** includes sleeve portion **462** connected to an outer surface of sample insert tube **405.** Sleeve portion **462** can be formed by thinning the tube wall of protective tube **460** and making step **437** at the inner surface of protective tube **460.** Sleeve portion **462** can be connected to the outer surface of sample insert tube **405** by any suitable mechanisms such as, for example, tight filing (e.g., pipe to pipe sleeve type fitting), or a threaded or ground joint.

**FIG.6** is a perspective view of an oxy-pyrohydrolysis reactor **510** including a sample insert **530,** according to any embodiments of the present disclosure. In some embodiments, sample insert **530** can be, for example, sample insert **330** of **FIG. 4****.** In some embodiments, sample insert **430** of **FIG. 5** may be used.

In the depicted embodiment of **FIG. 6****,** pyrotube **520** extends between first end **522** and second end **524** thereof. Pyrotube **520** includes combustion chamber **526,** and optional fluid inlets **542** and **546** configured to direct combustion ingredients such as, for example, oxygen and water into the pyrotube.

Sample insert **530** is connected to pyrotube **520** at first end **522** and configured to deliver samples into the combustion chamber **526.** Sample insert **530** includes sample insert tube **505** connected at junction **536.** As shown, outer opening **520** of sample insert tube **505** is positioned outside the pyrotube, while inner opening **540** extends a short distance through first end **522** into pyrotube **520.** In some embodiments, sample insert tube **505** can be a separate part removably sealed to pyrotube **520** at the junction **536.**

In some embodiments, sample insert tube **505** can be made of the same materials of the pyrotubes described herein. For example, both sample insert tube **505** and pyrotube **520** can be made of quartz or glass, which can be beneficial for forming the junction **536** to connect the parts.

Sample insert **530** further includes protective tube **560** connected to sample insert tube **505** at the inner opening **540** thereof. Second end **534** of protective tube **560** extends into combustion chamber **526** of the pyrotube to form the inner opening, through which samples can be delivered into the combustion chamber.

**FIG. 7** is a perspective view of another exemplary oxy-pyrohydrolysis reactor **610** including the sample insert **530.** Oxy-pyrohydrolysis reactor **610** is similar to oxy-pyrohydrolysis reactor **510** of **FIG. 6**, except oxy-pyrohydrolysis reactor **610** includes combustion-enhancing bed **652** disposed inside pyrotube **520.**

Although not shown, in some embodiments, oxy-pyrohydrolysis reactor **510** or **610** may further include a sheet of ceramic fabric disposed inside the pyrotube adjacent to the protective tube. Such a sheet of ceramic fabric can be, for example, sheet **54** of ceramic fabric or fiber disposed on a bottom of the pyrotube **20,** as shown in **FIG. 2B** and **FIG. 2C****.**

Various modifications and alterations of this invention will become apparent to those skilled in the art without departing from the scope of this invention which is defined by the appended claims.

## Claims

1. An oxy-pyrohydrolysis reactor (110) comprising:
a pyrotube (20, 320) comprising a combustion chamber positioned between a first end (22) and a second end (24) of the pyrotube; and
a sample insert (30, 330) connected to the first end (22) of the pyrotube (20), the sample insert (330) comprising:
a sample insert tube (305) having an inner opening (340) and an outer opening (320), wherein the sample insert tube (305) extends through the first end (22) of the pyrotube such that the inner opening (340) is located within the combustion chamber of the pyrotube a first distance X1 from the first end (22) of the pyrotube, and the outer opening (320) is located outside the pyrotube; and
a protective tube (360) having a first end connected to the inner opening (340) of the sample insert tube (305) and a second end (364) extending a second distance X2 from the first end (22) of the pyrotube into the combustion chamber of the pyrotube, wherein the ratio of X2 over X1 is at least 2;
wherein the sample insert tube (305) comprises a first material selected from quartz, glass, and combinations thereof; and the protective tube (306) comprises a second material selected from a ceramic, a metal, a metal alloy, and combinations thereof.

2. The oxy-pyrohydrolysis reactor of claim 1, wherein the first material comprises quartz and the second material comprises a ceramic.

3. The oxy-pyrohydrolysis reactor of claim 1 or 2, wherein the second material comprises one or more metals or metal alloys.

4. The oxy-pyrohydrolysis reactor according to any one of the preceding claims, wherein the ratio of X2 over X1 is at least 20.

5. The oxy-pyrohydrolysis reactor according to any one of the preceding claims, wherein the second end (364) of the protective tube (360) is configured to hold a sample comprising a solid, a liquid, an emulsion, or combinations thereof.

6. The oxy-pyrohydrolysis reactor according to any one of the preceding claims, wherein the second end (364) of the protective tube (360) is configured to deliver a fluid sample comprising a liquid or gas.

7. The oxy-pyrohydrolysis reactor according to any one of the preceding claims, further comprising a combustion-enhancing bed (50) comprising ceramic fibers or fabrics disposed inside the pyrotube, wherein the combustion-enhancing bed comprises at least one of randomly packed ceramic fibers and a roll of ceramic fabric.

8. The oxy-pyrohydrolysis reactor according to any one of the preceding claims, wherein the pyrotube further comprises at least one fluid inlet (42) (46) located at the first end of the pyrotube and a sheet (54) of ceramic fabric disposed inside the pyrotube near the at least one fluid inlet.

9. A combustion system (100) comprising a furnace (170) and the oxy-pyrohydrolysis reactor (110) according to any one of the preceding claims, wherein at least a portion of the oxy-pyrohydrolysis reactor is positioned inside the furnace.

10. The combustion system of claim 9, further comprising an analyzer (106) functionally connected to the furnace and configured to analyze a composition of combustion products produced in the pyrotube.

11. The combustion system of claim 10, wherein the analyzer (106) is configured to analyze the total fluorine content in the composition.

12. A method comprising:
delivering, via a sample insert (30, 330), a sample into a pyrotube (20) (320) from a first end (22) thereof, the sample containing one or more halogen elements, the sample insert (30, 330) connected to the pyrotube (20) at a junction thereof, the sample insert (30, 330) comprising:
a sample insert tube (305) having an inner opening (340) and an outer opening (320), wherein the sample insert tube (305) extends through the first end (22) of the pyrotube such that the inner opening (340) is located within the combustion chamber of the pyrotube (20) a first distance X1 from the first end (22) of the pyrotube (20), and the outer opening (320) is located outside the pyrotube; and
a protective tube (360) having a first end connected to the inner opening (340) of the sample insert tube (305) and a second end (364) extending a second distance X2 into the combustion chamber of the pyrotube (20), wherein the ratio of X2 over X1 is at least 2;
wherein the sample insert tube (305) comprises a first material selected from quartz, glass, and combinations thereof; and the protective tube (360) comprises a second material selected from a ceramic, a metal, a metal alloy, and combinations thereof; and
combusting the sample inside the pyrotube (20) to produce combustion products.

13. The method of claim 12 further comprising analyzing the combustion products to determine a total halogen content in the sample, optionally wherein the halogen in fluorine.

14. The method of claim 12 or 13, further comprising a combustion-enhancing bed (50) disposed inside the pyrotube (20) adjacent to a second end (24) of the pyrotube, the combustion-enhancing bed (50) comprising ceramic fibers or fabrics.

15. The method of any one of claims 12 to 14, wherein the combustion products comprise HF.

## Patentansprüche

1. Ein Oxypyrohydrolysereaktor (110), aufweisend:
ein Pyrorohr (20, 320), aufweisend eine Brennkammer, die zwischen einem ersten Ende (22) und einem zweiten Ende (24) des Pyrorohrs positioniert ist; und
einen Probeneinsatz (30, 330), der mit dem ersten Ende (22) des Pyrorohrs (20) verbunden ist, der Probeneinsatz (330) aufweisend:
ein Probeneinsatzrohr (305), das eine innere Öffnung (340) und eine äußere Öffnung (320) hat, wobei sich das Probeneinsatzrohr (305) durch das erste Ende (22) des Pyrorohrs derart erstreckt, dass sich die innere Öffnung (340) innerhalb der Brennkammer des Pyrorohrs um einen ersten Abstand X1 von dem ersten Ende (22) des Pyrorohrs befindet, und sich die äußere Öffnung (320) außerhalb des Pyrorohrs befindet; und
ein Schutzrohr (360), das ein erstes Ende, das mit der inneren Öffnung (340) des Probeneinsatzrohrs (305) verbunden ist, und ein zweites Ende (364) hat, das sich von dem ersten Ende (22) des Pyrorohrs in die Brennkammer des Pyrorohrs um einen zweiten Abstand X2 erstreckt, wobei das Verhältnis von X2 über X1 mindestens 2 ist;
wobei das Probeneinsatzrohr (305) ein erstes Material aufweist, das aus Quarz, Glas und Kombinationen davon ausgewählt ist; und das Schutzrohr (306) ein zweites Material aufweist, das aus einer Keramik, einem Metall, einer Metalllegierung und Kombinationen davon ausgewählt ist.

2. Der Oxypyrohydrolysereaktor nach Anspruch 1, wobei das erste Material Quarz aufweist und das zweite Material eine Keramik umfasst.

3. Der Oxypyrohydrolysereaktor nach Anspruch 1 oder 2, wobei das zweite Material ein oder mehrere Metalle oder Metalllegierungen aufweist.

4. Der Oxypyrohydrolysereaktor nach einem der vorstehenden Ansprüche, wobei das Verhältnis von X2 über X1 mindestens 20 beträgt.

5. Der Oxypyrohydrolysereaktor nach einem der vorstehenden Ansprüche, wobei das zweite Ende (364) des Schutzrohrs (360) konfiguriert ist, um eine Probe zu halten, aufweisend einen Feststoff, eine Flüssigkeit, eine Emulsion oder Kombinationen davon.

6. Der Oxypyrohydrolysereaktor nach einem der vorstehenden Ansprüche, wobei das zweite Ende (364) des Schutzrohrs (360) konfiguriert ist, um eine Fluidprobe abzugeben, aufweisend eine Flüssigkeit oder ein Gas.

7. Der Oxypyrohydrolysereaktor nach einem der vorstehenden Ansprüche, ferner aufweisend ein verbrennungsförderndes Bett (50), aufweisend Keramikfasern oder -gewebe, die innerhalb des Pyrorohrs angeordnet sind, wobei das verbrennungsfördernde Bett mindestens eines von zufällig gepackten Keramikfasern und einer Rolle aus Keramikgewebe aufweist.

8. Der Oxypyrohydrolysereaktor nach einem der vorstehenden Ansprüche, wobei das Pyrorohr ferner mindestens einen Fluideinlass (42) (46), der sich an dem ersten Ende des Pyrorohrs befindet, und eine Lage (54) eines Keramikgewebes aufweist, die innerhalb des Pyrorohrs nahe dem mindestens einen Fluideinlass angeordnet ist.

9. Ein Verbrennungssystem (100), aufweisend einen Ofen (170) und den Oxypyrohydrolysereaktor (110) nach einem der vorstehenden Ansprüche, wobei mindestens ein Abschnitt des Oxypyrohydrolysereaktors innerhalb des Ofens positioniert ist.

10. Das Verbrennungssystem nach Anspruch 9, ferner aufweisend einen Analysator (106), der mit dem Ofen funktionell verbunden und konfiguriert ist, um eine Zusammensetzung von Verbrennungsprodukten zu analysieren, die in dem Pyrorohr produziert werden.

11. Das Verbrennungssystem nach Anspruch 10, wobei der Analysator (106) konfiguriert ist, um den Gesamtfluorgehalt in der Zusammensetzung zu analysieren.

12. Ein Verfahren, aufweisend:
Abgeben, über einen Probeneinsatz (30, 330), einer Probe in ein Pyrorohr (20) (320) von einem ersten Ende (22) davon, wobei die Probe ein oder mehrere Halogenelemente enthält, wobei der Probeneinsatz (30, 330) an einer Verbindungsstelle davon mit dem Pyrorohr (20) verbunden ist, der Probeneinsatz (30, 330) aufweisend:
ein Probeneinsatzrohr (305), das eine innere Öffnung (340) und eine äußere Öffnung (320) hat, wobei sich das Probeneinsatzrohr (305) durch das erste Ende (22) des Pyrorohrs derart erstreckt, dass sich die innere Öffnung (340) innerhalb der Brennkammer des Pyrorohrs (20) um einen ersten Abstand X1 von dem ersten Ende (22) des Pyrorohrs (20) befindet, und sich die äußere Öffnung (320) außerhalb des Pyrorohrs befindet; und
ein Schutzrohr (360), das ein erstes Ende, das mit der inneren Öffnung (340) des Probeneinsatzrohrs (305) verbunden ist, und ein zweites Ende (364) hat, das sich in die Brennkammer des Pyrorohrs (20) um einen zweiten Abstand X2 erstreckt, wobei das Verhältnis von X2 über X1 mindestens 2 ist;
wobei das Probeneinsatzrohr (305) ein erstes Material aufweist, das aus Quarz, Glas und Kombinationen davon ausgewählt ist; und das Schutzrohr (360) ein zweites Material aufweist, das aus einer Keramik, einem Metall, einer Metalllegierung und Kombinationen davon ausgewählt ist; und Verbrennen der Probe innerhalb des Pyrorohrs (20), um Verbrennungsprodukte zu produzieren.

13. Das Verfahren nach Anspruch 12, ferner aufweisend das Analysieren der Verbrennungsprodukte, um einen Gesamthalogengehalt in der Probe zu bestimmen, optional wobei das Halogen in Fluor ist.

14. Das Verfahren nach Anspruch 12 oder 13, ferner aufweisend ein verbrennungsförderndes Bett (50), das innerhalb des Pyrorohrs (20) angrenzend an ein zweites Ende (24) des Pyrorohrs angeordnet ist, wobei das verbrennungsfördernde Bett (50) Keramikfasern oder -gewebe umfasst.

15. Das Verfahren nach einem der Ansprüche 12 bis 14, wobei die Verbrennungsprodukte HF aufweisen.

## Revendications

1. Réacteur d'oxypyrohydrolyse (110) comprenant :
un pyrotube (20, 320) comprenant une chambre de combustion positionnée entre une première extrémité (22) et une seconde extrémité (24) du pyrotube ; et
un insert d'échantillon (30, 330) relié à la première extrémité (22) du pyrotube (20), l'insert d'échantillon (330) comprenant :
un tube d'insert d'échantillon (305) ayant une ouverture interne (340) et une ouverture externe (320), dans lequel le tube d'insert d'échantillon (305) s'étend à travers la première extrémité (22) du pyrotube de telle sorte que l'ouverture interne (340) est située au sein de la chambre de combustion du pyrotube sur une première distance X1 depuis la première extrémité (22) du pyrotube, et l'ouverture externe (320) est située à l'extérieur du pyrotube ; et
un tube de protection (360) ayant une première extrémité reliée à l'ouverture interne (340) du tube d'insert d'échantillon (305) et une seconde extrémité (364) s'étendant sur une seconde distance X2 depuis la première extrémité (22) du pyrotube dans la chambre de combustion du pyrotube, dans lequel le rapport de X2 sur X1 est d'au moins 2 ;
dans lequel le tube d'insert d'échantillon (305) comprend un premier matériau choisi parmi le quartz, le verre, et des combinaisons de ceux-ci ; et le tube de protection (306) comprend un second matériau choisi parmi une céramique, un métal, un alliage métallique, et des combinaisons de ceux-ci.

2. Réacteur d'oxypyrohydrolyse selon la revendication 1, dans lequel le premier matériau comprend du quartz et le second matériau comprend une céramique.

3. Réacteur d'oxypyrohydrolyse selon la revendication 1 ou 2, dans lequel le second matériau comprend un ou plusieurs métaux ou alliages métalliques.

4. Réacteur d'oxypyrohydrolyse selon l'une quelconque des revendications précédentes, dans lequel le rapport de X2 sur X1 est d'au moins 20.

5. Réacteur d'oxypyrohydrolyse selon l'une quelconque des revendications précédentes, dans lequel la seconde extrémité (364) du tube de protection (360) est conçue pour contenir un échantillon comprenant un solide, un liquide, une émulsion, ou des combinaisons de ceux-ci.

6. Réacteur d'oxypyrohydrolyse selon l'une quelconque des revendications précédentes, dans lequel la seconde extrémité (364) du tube de protection (360) est conçue pour distribuer un échantillon de fluide comprenant un liquide ou un gaz.

7. Réacteur d'oxypyrohydrolyse selon l'une quelconque des revendications précédentes, comprenant en outre un lit d'amélioration de combustion (50) comprenant des fibres ou tissus céramiques disposés à l'intérieur le pyrotube, dans lequel le lit d'amélioration de combustion comprend au moins l'une des fibres céramiques emballées de manière aléatoire et un rouleau de tissu céramique.

8. Réacteur d'oxypyrohydrolyse selon l'une quelconque des revendications précédentes, dans lequel le pyrotube comprend en outre au moins une entrée de fluide (42) (46) située au niveau de la première extrémité du pyrotube et une feuille (54) de tissu céramique disposée à l'intérieur du pyrotube à proximité de l'au moins une entrée de fluide.

9. Système de combustion (100) comprenant un four (170) et le réacteur d'oxypyrohydrolyse (110) selon l'une quelconque des revendications précédentes, dans lequel au moins une partie du réacteur d'oxypyrohydrolyse est positionnée à l'intérieur du four.

10. Système de combustion selon la revendication 9, comprenant en outre un analyseur (106) connecté fonctionnellement au four et configuré pour analyser une composition de produits de combustion produits dans le pyrotube.

11. Système de combustion selon la revendication 10, dans lequel l'analyseur (106) est configuré pour analyser la teneur totale en fluor dans la com position.

12. Procédé comprenant :
la distribution, par l'intermédiaire d'un insert d'échantillon (30, 330), d'un échantillon dans un pyrotube (20) (320) depuis une première extrémité (22) de celui-ci, l'échantillon contenant un ou plusieurs éléments halogènes, l'insert d'échantillon (30, 330) étant relié au pyrotube (20) au niveau d'une jonction de celui-ci, l'insert d'échantillon (30, 330) comprenant :
un tube d'insert d'échantillon (305) ayant une ouverture interne (340) et une ouverture externe (320), dans lequel le tube d'insert d'échantillon (305) s'étend à travers la première extrémité (22) du pyrotube de telle sorte que l'ouverture interne (340) est située au sein de la chambre de combustion du pyrotube (20) sur une première distance X1 depuis la première extrémité (22) du pyrotube (20), et l'ouverture externe (320) est située à l'extérieur du pyrotube ; et
un tube de protection (360) ayant une première extrémité reliée à l'ouverture interne (340) du tube d'insert d'échantillon (305) et une seconde extrémité (364) s'étendant sur une seconde distance X2 dans la chambre de combustion du pyrotube (20), dans lequel le rapport de X2 sur X1 est d'au moins 2 ;
dans lequel le tube d'insert d'échantillon (305) comprend un premier matériau choisi parmi le quartz, le verre, et des combinaisons de ceux-ci ; et le tube de protection (360) comprend un second matériau choisi parmi une céramique, un métal, un alliage métallique, et des combinaisons de ceux-ci ; et la combustion de l'échantillon à l'intérieur du pyrotube (20) pour produire des produits de combustion.

13. Procédé selon la revendication 12 comprenant en outre l'analyse des produits de combustion pour déterminer une teneur totale en halogène dans l'échantillon, éventuellement dans lequel l'halogène dans le fluor.

14. Procédé selon la revendication 12 ou 13, comprenant en outre un lit d'amélioration de combustion (50) disposé à l'intérieur du pyrotube (20) à côté d'une seconde extrémité (24) du pyrotube, le lit d'amélioration de combustion (50) comprenant des fibres ou tissus céramiques.

15. Procédé selon l'une quelconque des revendications 12 à 14, dans lequel les produits de combustion comprennent de l'HF.
